# EUROPEAN PATENT SPECIFICATION

(11) **EP 1 743 670 B1**
(45) Date of publication and mention of the grant of the patent: **20.01.2010**
(21) Application number: 06019679.7
(22) Date of filing: 15.12.2000
(51) Int. Cl.: A61M 15/00, A61M 11/00, B65D 69/00, B65D 83/06, B05B 17/06

(54) **Device for treating packaged powders**
Vorrichtung zur Behandlung von verpackten Pulvern
Dispositif de traitement de poudres emballees

(30) Priority: 17.12.1999 US 172444 P
(43) Date of publication of application: 17.01.2007
(62) Divisional of application: 00989276.1
(73) Proprietor: Novartis AG, 4002 Basel (CH)
(72) Inventor: Schuler, Carlos, Cupertino, CA 95014 (US); Paboojian, Steve, Menlo Park, CA 94025 (US); Clark, Andrew, Half Moon Bay, CA 94062 (US); Rourke, Andrea, San Mateo, CA 94403 (US); Curley, Joanne, San Mateo, CA 94403 (US); Minami, Don S., Monte Sereno, CA 95030 (US); Tuttle, Derrick J., San Mateo, CA 94401 (US)
(74) Representative: Vossius & Partner

(56) References cited:
- EP-A- 0 277 708
- EP-A- 0 634 184
- WO-A-95/28192
- WO-A-97/25086
- WO-A-99/44663
- WO-A-99/64095
- US-A- 3 653 380
- US-A- 5 314 658
- US-A- 5 349 947
- US-A- 5 753 302
- US-A- 5 857 456
- US-A- 5 957 125
- US-A- 5 971 951

## Description

The invention is related generally to the field of powder extraction, and in particular to the extraction of powder from a receptacle to facilitate aerosolization of the powder. In one specific aspect, the invention is related to the conditioning of powder that is packaged within a receptacle to facilitate its removal during the aerosolization process.

One promising way to deliver various drugs to a patient is by pulmonary delivery where a drug dispersion or aerosol is inhaled by the patient so that the active drug within the dispersion can reach the distal or alveolar regions of the lung. Pulmonary drug delivery has shown to be particularly promising because certain drugs have been found to readily absorb within the blood circulation. For example, pulmonary delivery may be a useful approach for proteins and polypeptides that are difficult to deliver by other routes of administration.

A variety of techniques have been employed to deliver drugs to the lungs, including liquid nebulizers, metered dose inhalers, and the like. Of particular interest to the invention are dry powder dispersion devices that are able to aerosolize powdered medicaments for inhalation by the patient. Exemplary apparatus for aerosolizing powdered medicaments are described in U.S. Patent Nos. 5,458,135, 5,775,320, 5,740,794, 5,785,049, 6,089,228,6257233 and 6606992.

In WO 99/64095 a dry powder inhaler is disclosed comprising a first chamber in which a dry powder my be deaggregated by a vibrator and separated by size, and a second chamber in which the size-separated deaggregated powder may be picked by an air stream and carried for introduction into a patient.

Further, US-A-5 971 951 discloses a device for the generation of inhalable aerosols by extrusion through a porous membrane by a motor-driven cam assembly. The device holds a drug formulation package in a drug release position. Further a cam assembly is provided which is driven by motor so that a collapsible wall of the package is collapsed and the drug formulation is forced from the package through a porous membrane.

At least some of the apparatus described in the above references utilize a high pressure gas stream to draw the powder into a feed tube where the powder is deagglomerated, entrained in the high pressure gas stream, and exits as an aerosol suitable for inhalation. In some cases, such apparatus may utilize a receptacle that has a penetrable lid. The feed tube is inserted through the lid and one or more vents are formed in the lid. The high pressure gas stream then draws air through the receptacle and into the feed tube. The air drawn through the receptacle extracts the powder where it joins with the high pressure gas stream to form the aerosol.

As such, it is desirable to have the powder within the receptacle be in a condition that is suitable for extraction by a flowing gas stream. Exemplary techniques have been developed to fill the receptacle with the powder so that the powder is in a state conducive with being extracted in such a manner. Such techniques are described in, for example, U.S. Patent No. 5,826,633, and 6182712 and in PCT application no. 98/21059. Briefly, such techniques provide for the fluidization of the powder in the metering process. For example, the powder may be fluidized and then drawn into a meter chamber. The metered powder is then ejected into a receptacle that is then sealed with a lid.

Although these techniques have proven to be extremely successful in metering fine powders, due to a variety of circumstances, the powder in some cases may need to be further conditioned subsequent to packaging and prior to aerosolization. For example, in some cases, the powder may remain in the form of a puck, i.e., a loosely packed tablet, after being transferred from a metering chamber and into the receptacle. As other examples, conditions such as storage, bulk packaging, shipping, temperature, agitation, aging and the like may affect the condition of the powder within the receptacle. For instance, some of the powder may adhere to the sides of the receptacle, making it difficult to remove using the flowing gas stream. As another example, the powder may shift within the receptacle so that it is unevenly distributed. This may affect how the powder is extracted. As still another example, the powder may tend to agglomerate or clump together within the receptacle. As a further example, some of the powder may be caught or trapped in various corners that are formed within the receptacle.

Hence, the invention is related to techniques for conditioning pre-packaged powder to facilitate its removal from the packing. In this way, aerosolization of the powder may be facilitated.

The present invention is defined in the claims.

The invention provides techniques for treating or conditioning powders subsequent to their packaging to facilitate extraction of the powders from their packaging. The packaged powders will typically be fine, usually having an elementary particle size or mass median diameter in the range from about 0.1 µm to about 10 µm, preferably from about 0.5 µm to about 5 µm. Further, the powders may comprise various medicaments that have been metered and are configured to be aerosolized to facilitate pulmonary delivery.

A method can be used for conditioning a packaged powder. According to the method, a receptacle is provided having an enclosed chamber that contains an amount of powder. At least one pulse of energy is provided to the receptacle to increase the efficiency at which the powder may be extracted from the chamber when flowing a gas through the chamber.

One way to provide the pulse of energy is by quickly striking the receptacle, e.g., with at least about 1,1303 Nmm (0.01 lbf-in) of energy. For example, a spring-loaded lever may be released to quickly strike the receptacle. As another example, the receptacle may be moved past an arm that temporarily engages an edge of the receptacle as it passes the arm, or vice versa. Other ways to provide energy to the receptacle include flicking the receptacle with a finger, transferring the sudden energy resulting from the rupture of a fragile loaded structure, breaking a rigid element in the receptacle, bending and then releasing the receptacle to permit the receptacle to strike a surface, and the like.

Another way to provide the pulse of energy is by vibrating the receptacle. For example, a piezoelectric transducer may be placed against the receptacle and then vibrated, such as with a frequency of at least about 10 kHz. Alternatively, the receptacle may be placed into a sonic or ultrasonic bath to vibrate the receptacle. Other ways to vibrate the receptacle include coupling the receptacle to a vibrating structure, such as a tuning fork, vibrating a reed using the patient's inhalation, and the like.

The invention further provides a powder conditioning system that comprises a receptacle having an enclosed chamber containing an amount of powder. The system further includes a mechanism to provide at least one pulse of energy to the receptacle to increase the efficiency at which the powder may be extracted from the chamber when flowing a gas through the chamber.

In one aspect, the mechanism comprises a striking device to quickly strike the receptacle. The striking device may be configured to strike the receptacle with an energy of at least about 1,1303 Nmm (0.01 lbf-in) to condition the powder. As one example, the striking device may comprise a spring-loaded lever arm and a release apparatus to release the lever arm.

In another aspect, the mechanism_comprises an arm that is mounted to a frame. A movable platform is configured to hold the receptacle and to move the receptacle past the arm such that the arm temporarily engages the receptacle or vice versa. Alternatively, the mechanism may comprise a vibratable element that is configured to contact the receptacle when vibrating. For example, the vibratable element may comprise a piezoelectric transducer. As another example, a bending device may be used to bend and then release the receptacle to permit the receptacle to strike a surface.

In another embodiment, a powder dispersion device is provided that comprises a housing for receiving a receptacle having an enclosed cavity containing a powder. An aerosolization mechanism is provided in the housing to extract the powder from the receptacle and to form an aerosol. The dispersion device further includes a mechanism to provide at least one pulse of energy to the receptacle prior to aerosolizing the powder to facilitate removal of the powder from the receptacle when flowing a gas through the chamber.

In one aspect, the mechanism may comprise a striking device, such as a spring-loaded lever arm. In one particular aspect, a pivotal latch having a lock may be provided, with the latch pivoting as the receptacle is moved against the latch. A trigger having a ramp may also be provided, with the lock of the latch being slidable up the ramp when the latch pivots to cause the spring loaded lever arm to pivot and compress its spring. In so doing, the lock engages the trigger to lock the spring loaded lever arm in an energy storage position. Conveniently, another spring may be placed into contact with the trigger so that as the receptacle is further moved into the device, the receptacle engages and moves the trigger away from the lock to release the lever arm which then strikes the receptacle. Alternatively, the mechanism may comprise an arm extending from the housing to contact the receptacle, or a vibratable element to vibrate the receptacle.

A pre-conditioning method is provided for applying energy to the receptacles and/or coating the walls of the receptacle with the powder prior to applying at least one pulse of energy to the receptacle. For example, the pre-conditioning step may occur when the powder is being packaged in the receptacle. According to the method, the receptacle is subjected to low frequency vibration of less than about 1 kHz, and preferably about 0.01 - 500 Hz for a predetermined period of time sufficient to coat the receptacle with an amount of powder. Such pre-conditioning of the receptacle preferably takes place prior to the application of at least one pulse of energy by any of the methods discussed above.

The invention can be used in further a kit for aerosolizing a powder. The kit includes at least one receptacle having an enclosed chamber containing an amount of a powder. The kit further includes an aerosolization device to aerosolize the powder in the receptacle, and instructions describing a method for providing a pulse of energy to the receptacle prior to aerosolizing the powder.

The instructions describe a method for manually striking the receptacle with a finger or a hard surface. The kit further includes a powder conditioning device and instructions are provided describing techniques for placing the receptacle into the powder conditioning device prior to insertion into the aerosolization device. In one particular aspect, the powder conditioning device comprises a frame and a spring-loaded lever arm pivotally coupled to the frame. In this way, the lever arm may be released where it will pivot and strike the receptacle. Alternatively, the instructions may describe placement of the receptacle into the aerosolization device and operation of a button on the device to supply one or more pulses of energy to the receptacle prior to aerosolizing the powder.

In another embodiment, a powder within a sealed chamber of a receptacle may be conditioned when opening a container that holds the receptacle. Conveniently, such a container may be disposable and used as packaging when shipping or storing the receptacle. The container includes a mechanism to provide at least one pulse of energy to the receptacle to increase the efficiency at which the powder may be extracted from the chamber when flowing a gas through the chamber.

Conveniently, the container may comprise a base and a cover that is pivotally coupled to the base. The base and the cover define an enclosure for receiving the receptacle. Further, a coupling arrangement is provided to couple the receptacle to the base.

In one aspect, the mechanism comprises a hook that is coupled to the cover such that the hook engages and then releases the receptacle when the cover is pivoted to permit the receptacle to strike the base. In another aspect, the coupling arrangement may be pivotally coupled to the base, and the mechanism may comprise a latch that is operably coupled to the base, an arm that is coupled to the cover, and a cantilever beam that is attached to the coupling arrangement. The arm is configured to engage and pivot the coupling arrangement as the cover is opened, and the latch is configured to engage and then release the cantilever beam when the coupling arrangement is pivoted to permit the cantilever beam to strike the receptacle. Conveniently, the latch may be slidably coupled to the base such that the latch may be moved over the cantilever beam after the receptacle has been positioned within the enclosure. In still a further aspect, the mechanism may comprise a spring that is coupled to the cover and a latch that is operably coupled to the base. As the cover is closed, the spring engages the latch. The latch may then be operated to release the spring to permit the spring to strike the receptacle. Optionally, the latch may be slidably coupled to the base such that the latch may be moved to release the spring after the cover has been closed.

An alternative embodiment provides a container or housing for holding multiple receptacles. Prior to removal of one of the receptacles from the container, a pulse of energy is supplied to the receptacle to condition the powder. In this way, multiple receptacles may be stored within a single housing a dispensed upon demand. Prior to dispensing the powder is conditioned to facilitate its aerosolization in an aerosolization apparatus.

The powder may be conditioned by stacking the receptacles within the container and striking the top receptacle before it is ejected from the container. This may be accomplished by using a biased striking member and a trigger that is movable between a home position and a striking position. As the trigger is moved to the striking position, the striking member is released to permit the striking member to strike a top one of the receptacles. Just prior to releasing the striking member, an advancement apparatus advances the receptacles toward the striking member. In this way, after the powder is conditioned the trigger may be moved back to the home position where a push plate that is coupled to the trigger pushes the treated receptacle from the container.

### BRIEF DESCRIPTION OF THE DRAWINGS

Fig. 1 is a bottom perspective view of one embodiment of a receptacle for holding a powder according to the invention.
Fig. 2 is a graph illustrating the percentage of powder remaining within a receptacle after being exposed to a high pressure gas stream of an aerosolizing apparatus. The receptacles include those that have been filled only (control), receptacles that have been filled and then shipped, and receptacles that have been filled, then shipped, and then treated with a striking device.
Fig. 3 is a graph illustrating the emitted dose from receptacles to which different amounts of energy were previously applied by a striking device. The receptacles were either filled only (control) or filled and then included in a standard shipping procedure.
Fig. 4 is a graph illustrating the different energy levels used in the trial of Fig. 3.
Fig. 5 is a graph illustrating the result of increasing energy levels when striking receptacles containing a certain powder during a conditioning process.
Fig. 6 is a graph illustrating the result of increasing the amount of vibration energy supplied to receptacles containing a certain powder during a conditioning process.
Fig. 7 is a top perspective view of a powder conditioning device and a receptacle inserted into the device.
Figs. 7A-7E illustrate operation of the powder conditioning device of Fig. 7 to condition a powder within the receptacle.
Fig. 8 is a cross-sectional side view of a powder conditioning device that may be included with a aerosolization apparatus.
Fig. 9 is a perspective view of an aerosolization apparatus that may utilize the powder conditioning device of Fig. 8.
Fig. 10 illustrates a powder conditioning device using vibratory energy to condition the powder.
Fig. 11 illustrates an alternative embodiment of the device of Fig. 10.
Figs. 12A-12C schematically illustrate the use of an alternative powder conditioning scheme.
Fig. 13 illustrates an embodiment of a powder conditioning device.
Fig. 14 is an exploded view of the device of Fig. 13.
Figs. 15-22 illustrate operation of the powder conditioning device of Fig. 13 to condition powder within a receptacle.
Fig. 23 is a perspective view of another embodiment of a powder conditioning device.
Fig. 24 illustrates the powder conditioning device of Fig. 23 in operation.
Fig. 25 is a perspective view of a further embodiment of a powder conditioning device.
Fig. 26 illustrates the powder conditioning device of Fig. 25 in operation.
Fig. 27 is a perspective view of still another embodiment of a powder conditioning device.
Fig. 28 illustrates the powder conditioning device of Fig. 27 when in a open position.
Fig. 29 illustrates an embodiment of a powder conditioning device for holding multiple receptacles .
Figs. 30 and 31 illustrate operation of the powder conditioning device of Fig. 29.

### DESCRIPTION OF THE SPECIFIC EMBODIMENTS

The invention provides for the treatment or conditioning of packaged powder prior to extraction from a receptacle or container. The powder conditioning techniques of the invention may be utilized in connection with essentially any type of receptacle or container that is employed to hold the powder. Merely by way of example, the invention may be utilized with "blister packs" or receptacles that have a sealed cavity in which the powder is held. Examples of such receptacles are described in U.S. Patent No. 5,740,794. However, it will be appreciated that the invention is not limited to these specific receptacles.

The invention is particularly useful in conditioning fine powders. For example, the invention may be used with powders having a mean particle size in the range from about 0.1 µm to about 10 µm, preferably from 0.5 µm to about 5 µm, and more preferably from about 1 µm to about 4 µm. A wide variety of powders may be conditioned using the techniques of the invention including, for example, active agents that include but are not limited to calcitonin, erythropoietin (EPO), Factor VIII, Factor IX, ceredase, cerezyme, cyclosporin, granulocyte colony stimulating factor (GCSF), alpha-1 proteinase inhibitor, elcatonin, granulocyte macrophage colony stimulating factor (GMCSF), growth hormone, human growth hormone (HGH), growth hormone releasing hormone (GHRH), heparin, low molecular weight heparin (LMWH), interferon alpha, interferon beta, interferon gamma, interleukin-2, luteinizing hormone releasing hormon (LHRH), insulin, somatostatin, somatostatin analogs including octreotide, vasopressin analog, follicle stimulating hormone (FSH), insulin-like growth factor, insulintropin, interleukin-1 receptor antagonist, interleukin-3. interleukin-4. interleukin-6. macrophage colony stimulating factor (M-CSF), nerve growth factor, parathyroid hormone (PTH), thymosin alpha 1, llb/lla inhibitor, alpha-1 antitrypsin, VLA-4, respiratory synchtial virus antibody, cystic fibrosis transmembrane regulator (CFTR) gene, deoxyribonuclease (Dnase), bactericidal/permeability increasing protein (BPI), anti-CMV antibody, interleukin-1 receptor, 13-cis retinoic acid, pentamidine isethiouate, albuterol sulfate, metaproterenol sulfate, beclomethasone diprepionate, triamcinolone acetamide, budesonide acetonide, gentamicin, ciprofloxacin, tobramycin, fluticasone, ipratropium bromide, flunisolide, cromolyn sodium, ergotamine tartrate and the analogues, agonists and antagonists of the above.

When filling the receptacles with powder, the powder may be metered. For example, with powdered medicaments, a metering process may be employed to meter a unit dose of a medicament. Examples of such metering processes are described in U.S. Patent Nos. 5,826,633, and 6182712 and PCT Application No. 98/21059. However, it will be appreciated that the invention is not intended to be limited to any specific filling process. When metering the powder, unit dosages in the range from about 0.5 mg to about 40 mg may be placed may be placed within the receptacles, it being appreciated that the invention may be used to condition other amounts of powder.

The powder may be extracted or removed from the receptacles by a vacuum that is created within the receptacle. In creating the vacuum, air or another gas flows through the receptacle, assisting in the removal of the powder. For example, one way to remove the powder is to provide at least one exit opening and one or more vents in the receptacle. The powder may then be drawn out of the exit, with air entering the vent and sweeping through the cavity or chamber holding the powder. Examples of how powder may be extracted from receptacles as well as aerosolization apparatus suitable for use with the methods according to this invention are described in U.S. Patent Nos. 5,740,794 5,785,049, and 6089228.

The powder conditioning techniques of the invention may be useful in conditioning powder that is in a variety of states. For example, the powder may be agglomerated into large agglomerates, densely packed, unevenly distributed, adhered to the walls of the receptacle, and the like. Such powder states may arise due to conditions such as agitation, shipping, packaging, initial filling, temperature, passage of time, the type of powder, and the like.

According to the invention, one or more energy pulses are provided to the receptacles to treat or condition the powder. For example, a quick strike or "flick" may be provided to the receptacle. This may be accomplished, for example, by releasing a spring-loaded lever arm or a flexible member, by flicking with a finger, by moving the receptacle past an arm that engages the receptacle (or vice versa), by transferring the sudden energy resulting from the rupture of a fragile loaded structure, by breaking a rigid element in the receptacle, by propelling a solid object (such as a ball bearing) against the receptacle, by bending and then releasing the receptacle to permit the receptacle to strike a surface, and the like. Vibratory energy, such as sonic or ultrasonic vibrations, may also be supplied to the receptacle. Vibratory energy may be supplied using, for example, a piezoelectric transducer, a sonic bath, a vibrating structure such as a vibrating reed, a tuning fork, and the like. Other techniques for supplying energy to the powder include an air pulse, a sudden rupture of the receptacle, a breaking of a rigid element of the receptacle, or a separation of the receptacle from a web of a receptacles in such a way that a sharp impulse is propagated through the package.

The powder may be conditioned prior to insertion of the receptacle into an aerosolization apparatus, after insertion, or both. Conveniently, the aerosolization apparatus may be constructed to condition the powder during normal operation, making the processes invisible to the end user. In some cases, the receptacles may be part of a kit that includes instructions on how to condition the powder prior to aerosolization. For example, the instructions may describe techniques for treating the receptacle prior to insertion into an aerosolization apparatus. Alternatively, the instructions may describe operation of an aerosolization device to condition the powder prior to aerosolization. As another option, the powder may be conditioned upon removal of the receptacle from a container of package in which the receptacle is stored.

According to a preferred embodiment, the conditioning event is synchronized with the aerosolization event. It has surprisingly been found that this timing of the conditioning event results in improved emitted doses from the delivery device. According to a particularly preferred embodiment, synchronization is performed by providing that the conditioning event occurs about 100 msec before up to about 25 msec after initiation of the aerosolization event. Emitted doses using such synchronization have been observed to be greater by more than 10% of emitted doses where conditioning and aerosolization were not synchronized.

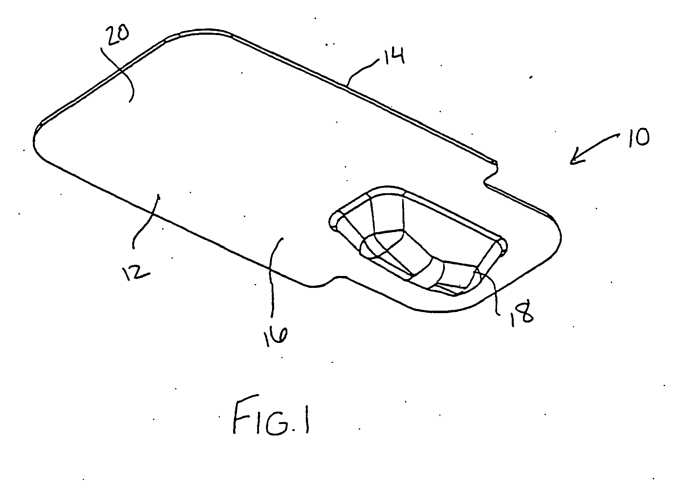

Referring now to Fig. 1, one embodiment of a receptacle 10 will be described to illustrate the powder conditioning techniques of the invention. In so doing, it will be appreciated that the techniques of the invention may be used with other types of receptacles. Receptacle 10 comprises a receptacle body 12 having a top end 14 and a bottom end 16. Receptacle body 12 defines a cavity 18 for holding an amount of powder. A tab 20 extends from cavity 18 and provides a convenient way to hold and handle receptacle 10. Receptacle body 12 may be constructed from a variety of rigid or semirigid materials, including metals, plastics, composites, and the like.

One convenient way to extract the powder from cavity 18 is to create an exit opening and one or more vents in top end 14. Suction may then be provided to draw air through the vent, through cavity 18 and out the exit opening.

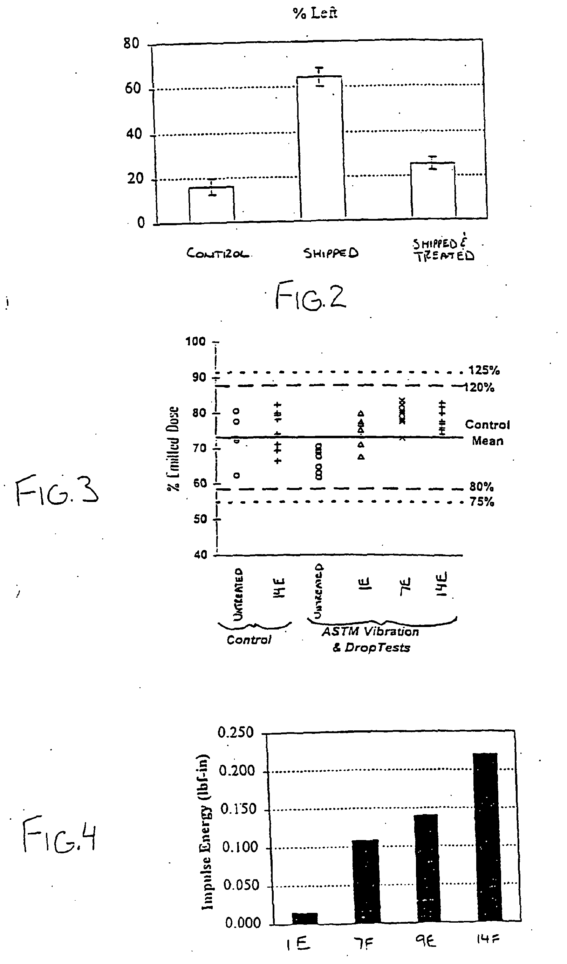

To condition the powder in cavity 18, one or more pulses of energy may be provided to receptacle body 12. The effectiveness of conditioning the powder in this manner to facilitate extraction of the powder using a flow of air is illustrated in Fig. 2. Fig. 2 graphically illustrates the results of a trial where three groups of receptacles were evaluated. The first group is a control group where the powder was extracted immediately after filling. The second group represents a group of receptacles after being subjected to conditions typically experienced when shipping the receptacles. The third group represents receptacles that have experienced shipping conditions and have then been conditioned by applying a pulse of energy to the receptacle.

As shown, the amount of powder remaining in the group of shipped receptacles exceeded 60%, with a relatively large deviation from the mean. After conditioning, the amount of powder extracted was similar to the control group, with a deviation significantly smaller than the shipped group. In some cases, the treated group may have a smaller deviation from the mean than the control group.

Fig. 3 is a graph illustrating test results from another trial. The trial of Fig. 3 utilized a control group and a processed group. The control group included receptacles where the powder was just introduced. The processed group was subjected to ASTM vibration and drop tests to simulate a shipping situation. The receptacles were then subjected to various impulses or snaps. The term "snap" indicates that the receptacle was quickly struck or "snapped." Fig. 4 illustrates the amount of energy supplied for each group and includes a base snap, 1 * E, where E = 1,8085 Nmm (0.016 lbf-in), a 7 * E snap, a 9 * E snap, and a 14 * E snap.

Fig. 3 illustrates that the increase in impulse energy increased the amount of powder that was extracted from the receptacle. Further, for the 14 * E snap after processing, the deviation from the mean was significantly reduced.

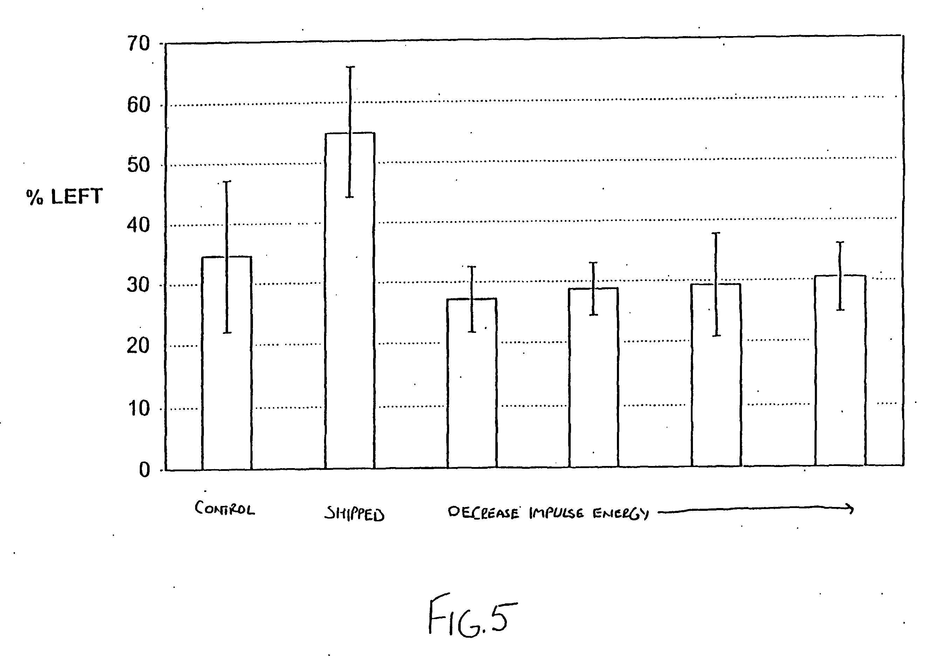

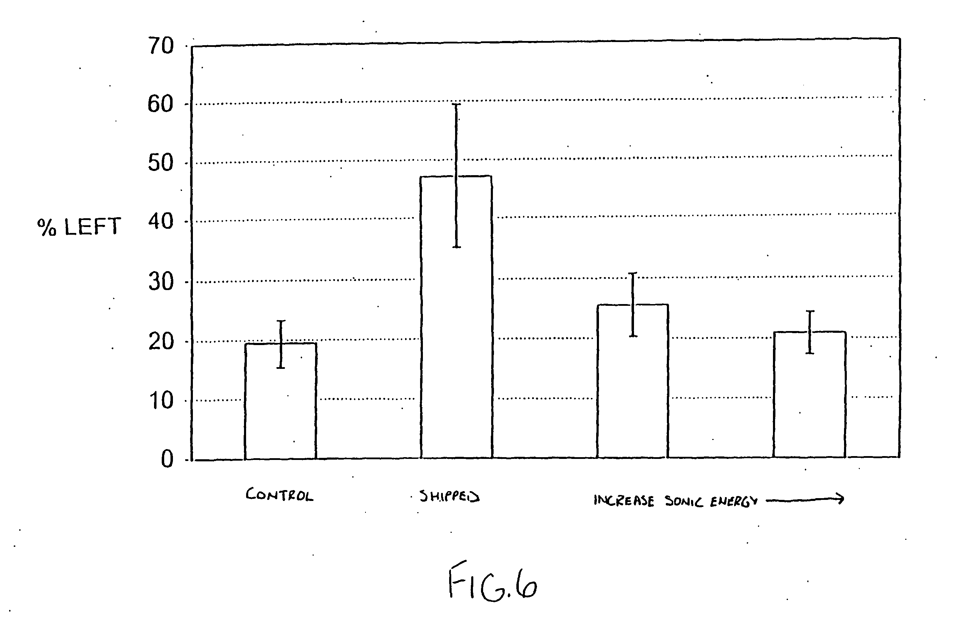

Fig. 5 is another graph illustrating the increase in powder removal efficiency as the striking impulse to a receptacle is increased for a certain powder after being subjected to shipping conditions. Fig. 6 illustrates a similar graph where vibratory energy was applied to the receptacles. As shown, when increasing the time at which sonic energy was applied, the amount of powder extracted was increased. Further, the deviation from the mean was also reduced.

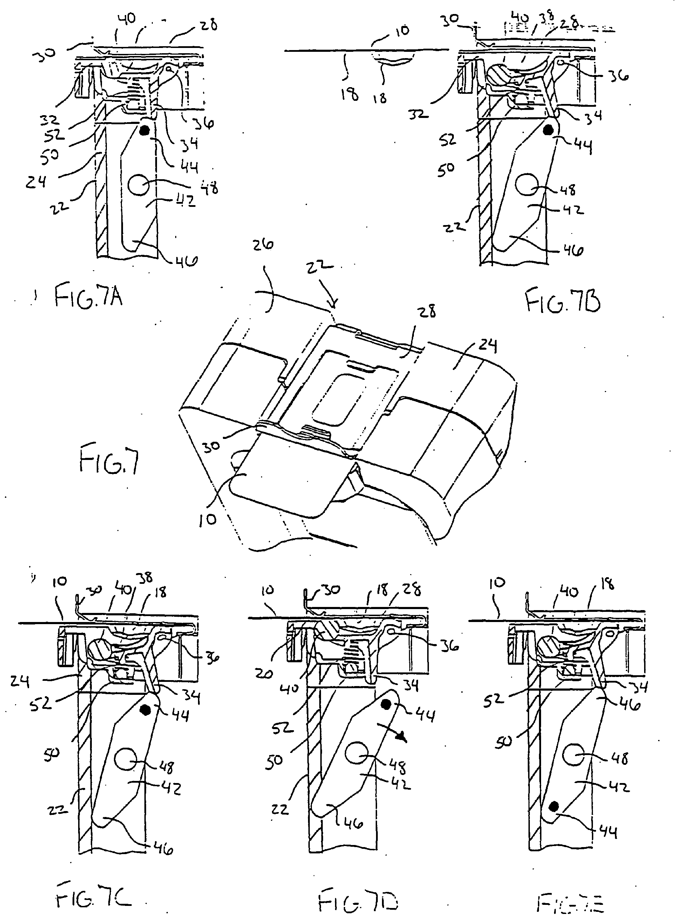

Fig. 7 illustrates an embodiment of a powder conditioning device 22. For convenience of discussion, device 22 is shown in connection with receptacle 10 of Fig. 1, it being appreciated that other types of receptacles may also be used. Device 22 comprises a frame 24 to which various components are coupled. At the top end 26 of frame 24 is a guide member 28 having a curved lip 30 to facilitate introduction of receptacle 10. Frame 24 further includes an opening 32 (see Fig. 7A) disposed below guide member 28 through which receptacle 10 may be inserted. Referring also to Fig. 7A (where receptacle 10 has been removed), device 22 further includes a lever arm 34 that is pivotally attached to frame 24 at a pivot point 36. Lever arm 34 includes a recess 38 for receiving cavity 18 of receptacle 10. A cylindrical striking member 40 is employed to strike receptacle 10 when device 22 is operated.

A rotatable trigger 42 having two ends 44 and 46 is pivotally coupled to frame 24 at a pivot point 48. When rotated, ends 44 engage lever arm 34 as described hereinafter. Frame 24 further includes holder 50 that holds a spring 52 that engages lever arm 34. In this way, lever arm 34 is biased in a closed position as shown in Fig. 7A. To insert receptacle 10, trigger 42 is rotated slightly to the position shown in Fig. 7B. This causes lever arm 34 to pivot and begin to compress spring 52. Also, cylindrical striking member 40 is moved downward to permit cavity 18 of receptacle 10 to be received within device 22 as shown in Fig. 7C. Further rotation of trigger 42 causes spring 52 to further compress as shown in Fig. 7C. At a certain point, end 44 of trigger 42 moves past lever arm 34 as shown in Fig. 7D. At this point, spring 52 forces lever arm 34 back to the closed position where striking member 40 engages receptacle 10 on a side of cavity 18 and at tab 20 just proximal to cavity 18 as shown in Fig. 7D. When striking receptacle 10, an impulse of energy is provided to condition the powder within cavity 18.

As shown in Fig. 7E, trigger 42 is_rotated about 180 degrees to where end 46 contacts lever arm 34. In this way, striking member 40 may be lowered to permit receptacle 10 to be removed from device 22. Receptacle 10 may then be inserted into an aerosolization device to aerosolize the powder prior to inhalation.

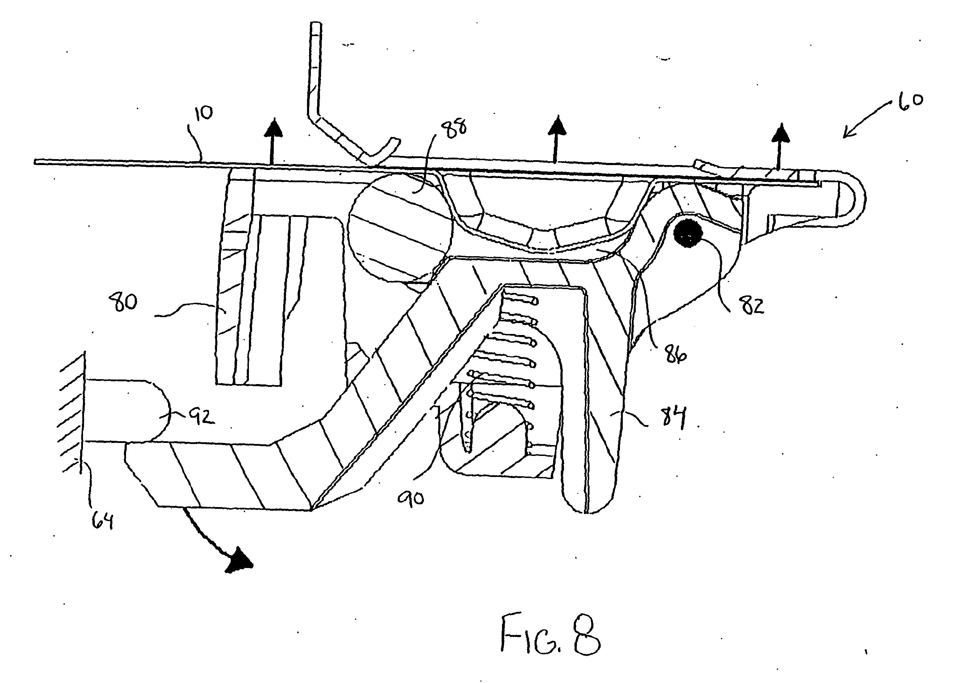

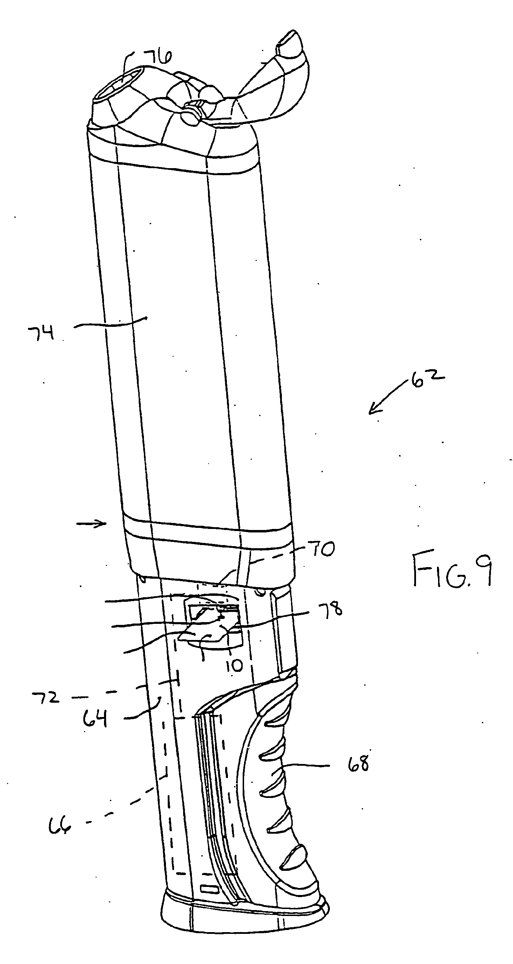

Fig. 8 illustrates a striking mechanism 60 that may be incorporated into an aerosolization apparatus, such as aerosolization apparatus 62 of Fig. 9. Aerosolization apparatus 62 may be configured in many respects to be similar to the aerosolization devices described in U.S. Patent No. 5,740,794. However, it will be appreciated that the invention is not intended to be limited to using striking mechanism 60 only with apparatus 62.

Apparatus 62 comprises a base 64 in which striking mechanism 60 is housed along with a piston pump 66 for producing a pressurized gas using a handle 68. An aerosolizing mechanism 70 receives the pressurized gas from piston pump 66 via tubing 72 to extract and aerosolize powder from receptacle 10. The aerosolized powder is ejected into a capture chamber having a mouthpiece 76 for inhaling the powder. Base 64 also includes an opening 78 into which receptacle 10 is inserted.

When receptacle 10 is inserted into opening 78, receptacle 10 is received into a receptacle holder 80 (see Fig. 8). A thumb-toggle extending from base 64 (hidden from view) may be depressed to move receptacle holder 80 upward. In so doing, receptacle 10 is moved upward within opening 78 until being coupled with aerosolization mechanism 70. A fire button 80 may then be operated to release the pressurized gas into aerosolization mechanism 70 which aerosolizes the powder within receptacle 10 and ejects the powder into capture chamber 74.

Referring back to Fig. 8, construction of striking mechanism 60 will be described. Pivotally coupled to receptacle holder 80 at a pivot point 82 is a lever arm 84. A cavity 86 is formed in lever arm 84 for receiving receptacle 10. Further, a striking member 88 is provided to strike receptacle 10 and condition its powder. A spring 90 is positioned between holder 80 and lever arm 84 to bias lever arm 84 in the position shown in Fig. 8.

Extending from base 64 (see also Fig. 9) is a projection 92 that engages lever arm 84 as striking mechanism 60 is moved upward as shown by the arrows. Conveniently, the thumb toggle of aerosolizing apparatus 62 may be depressed to lift striking mechanism 60. In so doing, lever arm 84 is pivoted as it engages projection 92. Consequently, spring 90 is compressed. At a certain point, lever arm 84 moves past projection 92 where spring 90 forces striking member 88 against receptacle 10 to condition the powder. Further movement of the thumb toggle engages receptacle 10 with aerosolization mechanism 70 (see Fig. 9) and releases the pressurized gas to extract the powder from receptacle 10 as previously described. In this way, conditioning of the powder occurs as part of the aerosolization process and is invisible to the end user.

The force applied by striking mechanism 60 may be varied depending on the nature of spring 90 and the amount of spring compression. In one aspect, striking mechanism 60 may be configured to produce a striking energy in the range from about 1,1303 Nmm (0.01 lbf in) to about 33,91 Nmm (0.3 lbf-in).

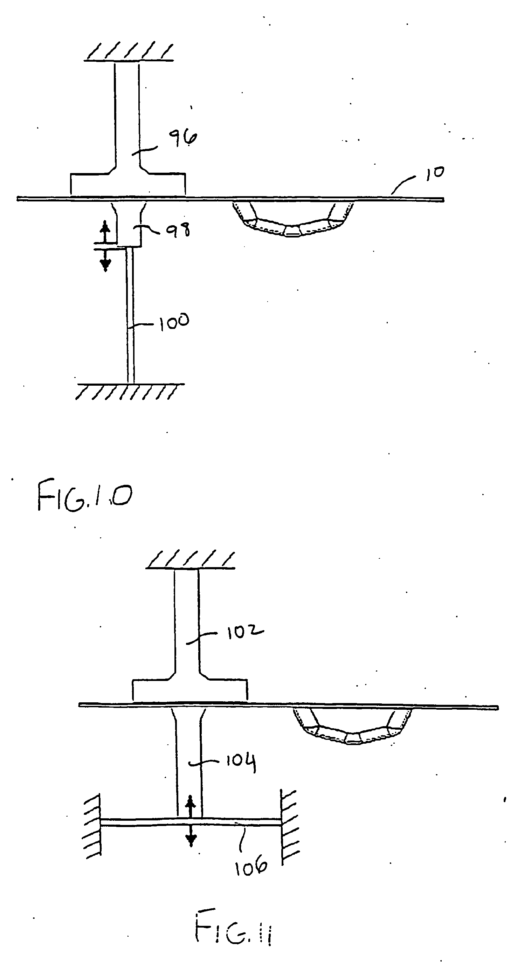

Another way to condition the powder within receptacle 10 is to use vibratory energy. Two examples of how vibratory energy may be supplied are illustrated in Figs. 10 and 11. In Fig. 10, receptacle 10 is clamped between two clamping members 96 and 98, with clamp 96 being held stationary as shown. A piezoelectric rod 100 is positioned against clamp 98 and is fixed at its opposite end as shown. When a voltage is applied to rod 100, it lengthens and shortens as shown by the arrows. The resulting displacement of clamp 98 is small with a high oscillating force. For example, the oscillating power may be in the range from about 10 milliwatts to about 50 milliwatts. Further, rod 98 may be vibrated for a time in the range from about 0.5 sec to about 10 sec to condition the powder in receptacle 10.

In Fig. 11, receptacle 10 is clamped between a pair of clamps 102 and 104. Clamp 102 is fixed at one end as shown. Contacting clamp 104 is a circular piezoelectric member 106 that is fixed or clamped about its periphery as shown. When a voltage is applied, piezoelectric member 106 vibrates up and down as shown to oscillate clamp 104 against receptacle 10.

The schemes shown in Figs. 10 and 11 may conveniently be incorporated into an aerosolization apparatus. In such cases, the clamping members may be configured to clamp the receptacle after its insertion into the apparatus. For example, the apparatus may be configured to automatically clamp the receptacle and provide the vibratory energy upon insertion of the receptacle. Alternatively, the apparatus may include a button that is operated by the user to clamp the receptacle. In either case, the apparatus may include a processor to control the application of the voltage. In this way, the processor may be programmed so that the receptacle is vibrated for a certain time upon each operation.

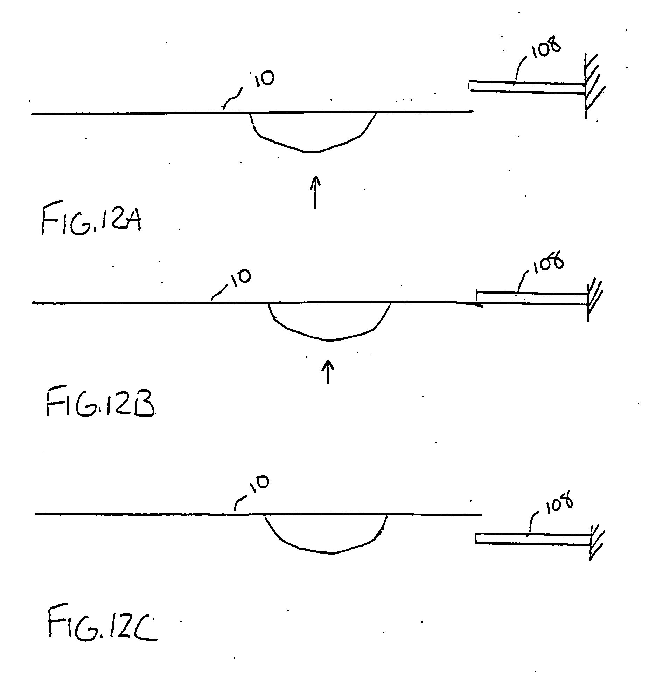

Figs. 12A-12C illustrate another scheme for providing a pulse of energy to receptacle 10. This scheme involves the use of a projection 108 that may be included as part of an aerosolization apparatus or a separate conditioning device. Initially, receptacle 10 is positioned below projection 108 as shown in Fig. 12A. Receptacle 10 is then moved upward until engaging projection 108 as shown in Fig. 12B. This upward movement may conveniently be a part of the process where receptacle is moved into an aerosolization mechanism in a manner similar to that previously described. As receptacle 10 is moved past projection 108 (see Fig. 12C), receptacle 10 is abruptly released to vibrate receptacle 10 and condition the powder.

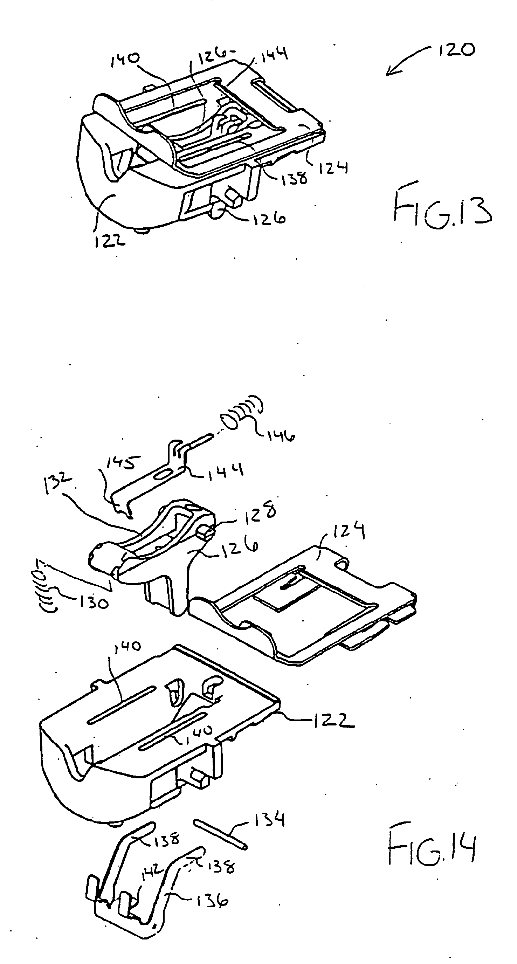

Figs. 13 and 14 illustrate another embodiment of a powder conditioning device 120. As shown, device 120 is a stand alone system. However, it will be appreciated that device 120 may be incorporated into a powder dispersion device in a manner similar to that described with previous embodiments.

Device 120 comprises a carrier 122 that serves as a carrier or housing for the other components of device 120 and provides a convenient structure to permit device 120 to be incorporated into a powder dispersion device. A retainer 124 is positioned on top of carrier 122 to facilitate insertion of a receptacle into carrier 122.

Device 120 further includes an arm 126 that is pivotally coupled to carrier 122 at a pivot point 128. A compression spring 130 is employed to bias arm 126 toward retainer 124. Arm 126 includes a recessed region 132 into which a cavity of a receptacle (not shown) is received when fully inserted into carrier 122 as described hereinafter. Also pivotally coupled to carrier 122 by a pin 134 is a latch 136 having a pair of arms 138 that may move within a pair of slots 140 in carrier 122 when latch 136 is pivoted. Latch 136 also includes a lock 142 that is employed to hold arm 126 in an energy storage position as described hereinafter. A trigger 144 is also provided to permit arm 126 to be released from the energy storage position as described hereinafter. Trigger 144 includes a ramp 145 over which lock 142 may slide. A spring 146 is employed to laterally bias trigger 144.

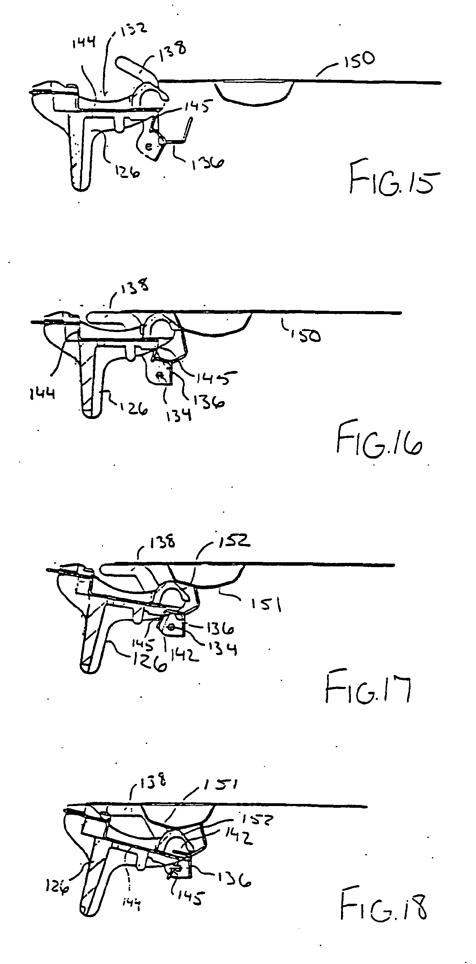

Referring now to Figs. 15-22, operation of device 120 to condition the powder within a receptacle 150 will be described. As receptacle 150 is inserted into carrier 122, it engages arms 138 of latch 136 (see Fig. 15). Further insertion of receptacle 150 causes latch 136 to pivot as shown in Fig. 16 preparing lock 142 to catch trigger 144. A tub 151 of receptacle 150 contacts a ramp 152 of arm 126 causing arm 126 to pivot (see Fig. 17) against the bias of spring 130 (see Fig. 14). As shown in Fig. 18, lock 142 moves over trigger 144 to lock arm 126 in an energy storage position where spring 130 is compressed.

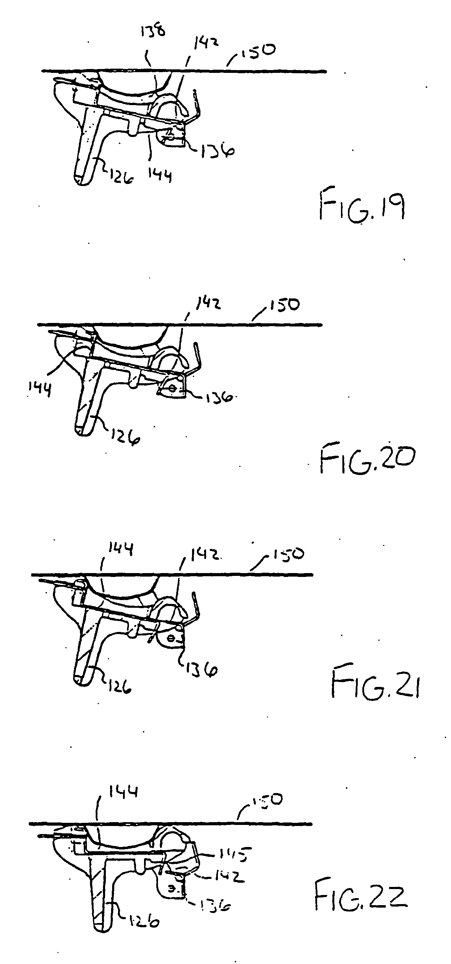

As receptacle 150 is further inserted into carrier 122, receptacle 150 contacts trigger 144 as shown in Fig. 19 and slides trigger 144 against the bias of spring 146 (see Fig. 14) and away from lock 142 as shown in Fig. 20. As receptacle 150 is further inserted, lock 142 clears trigger 144 (see Fig. 21) and arm 126 is quickly struck against receptacle 150 due to the force created by spring 130 as shown in Fig. 22. In the position of Fig. 22, arm 126 and carrier 122 lock receptacle 150 in place to permit the powder from receptacle 150 to be extracted and aerosolized while receptacle 150 is secured in place.

According to another embodiment, a receptacle may be subjected to low frequency vibration prior to application of at least one pulse of energy, such as during packaging of the receptacle and prior to shipping. For example, the receptacle may be vibrated on a vibration table or fixture at a frequency of less than about 1 kHz, preferably from about 0.01 Hz to about 500 Hz, and more preferably from about 1 Hz to about 150 Hz for a period of time sufficient to coat the receptacle with an amount of powder, typically up to about 10 minutes, preferably within about 0.01 to about 10 minutes. Other techniques for applying low frequency vibration include use of an audio speaker, a metal engraver, and the like This low frequency vibration pre-conditions the powder within the receptacle by coating the walls of the receptacle with an amount of powder. Subsequent application of at least one pulse of energy by any of the methods discussed above to such pre-conditioned receptacles results in even greater dispersion of powder from the receptacle.

In another aspect of the invention, powders that are packaged within receptacles may be conditioned upon removal from a container or other packaging material in which the receptacles are stored. In this way, receptacles may be placed within a package or other container at the time of manufacture as is common within the art. However, the package may include a mechanism for conditioning the powder within the receptacle upon removal of the receptacle from the package. In this way, a disposable receptacle package may be used to both store and ship the receptacle as well as to condition the powder prior to placement of the receptacle into an aerosolization device. Use of such a package is advantageous in that it ensures the powder will be conditioned prior to aerosolization.

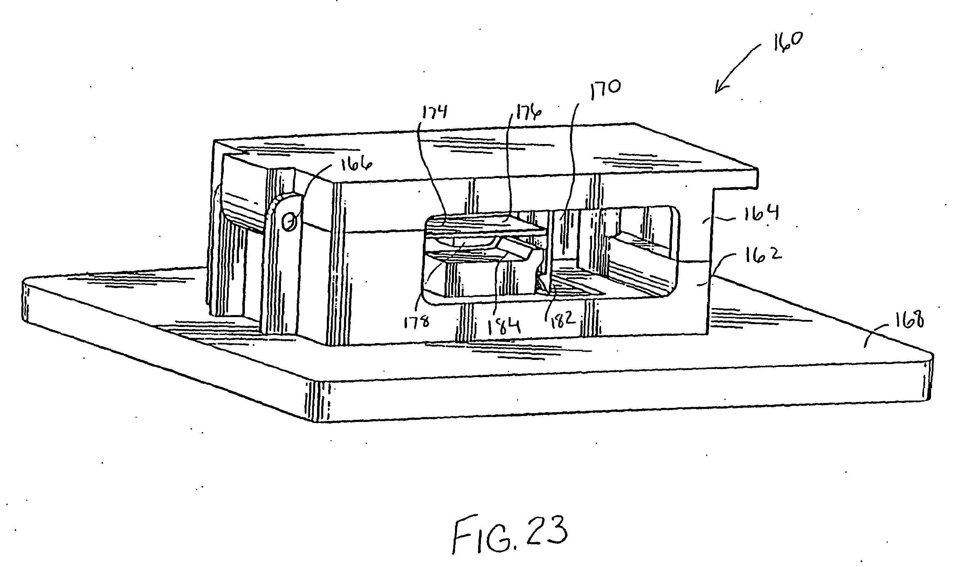

Referring now to Fig. 23, one embodiment of such a powder conditioning device 160 will be described. Device 160 comprises a base 162 and a cover 164 that is pivotally coupled to base 162 by a hinge 166. Optionally, base 162 may be coupled to a platform 168. As shown in Fig. 23, device 160 is in a closed position where base 162 and cover 164 define an enclosure 170.

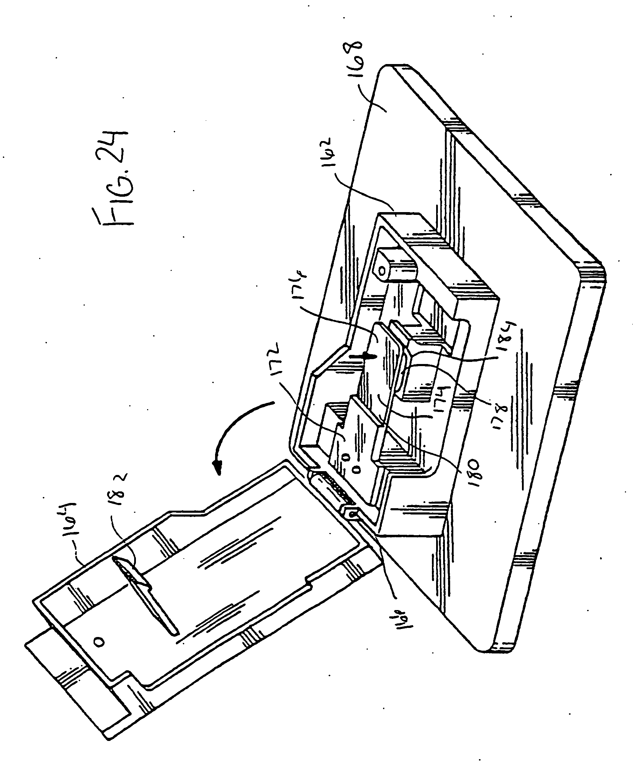

As best shown in Fig. 24, a coupling arrangement 172 is employed to couple a receptacle 174 to base 162. Receptacle 174 has a top end 176 and a cavity 178 positioned beneath top end 176 which is filled with a powder that is to be conditioned. A tab 180 extends from cavity 178 and is coupled to coupling arrangement 172. Conveniently, tab 180 may be scored so that it may be removed from coupling arrangement 172 when ready to be inserted into an aerosolization device. Conveniently, coupling arrangement 172 may include location pins to properly position receptacle 174 within enclosure 170. Further, a viewing window may be provided to permit the contents of enclosure 170 to be visualized.

Coupled to cover 164 is a hook 182. When cover 164 is moved to an open position as shown in Fig. 24, hook 182 engages receptacle 174 to move receptacle 174 upward. As cover 164 is further opened, receptacle 174 moves past hook 182 and receptacle 174 springs downward until engaging a protruding surface 184 of base 162. As cavity 178 strikes surface 184, energy is supplied to the powder held in cavity 178 to condition the powder. The amount of energy supplied to the powder may be varied by changing the characteristics or thickness of the material used to construct receptacle 174. The energy supplied to the powder may further be varied by adjusting the distance between receptacle 174 and surface 184 as well as the size of hook 182.

Hence, receptacle 174 may be placed into enclosure 170 at the time of manufacture. Device 160 may then function as a package for shipping or storing receptacle 174 until ready for use. At such time, cover 164 is lifted to gain access to receptacle 174. In so doing, the powder within receptacle 174 is conditioned as previously described. Receptacle 174 may then be removed from enclosure 170 and placed in an aerosolization device for aerosolization of the powder. Although not shown, it will be appreciated that powder conditioning device 160 may be modified to hold multiple receptacles so that the powder within each receptacle is simultaneously conditioned when cover 164 is opened.

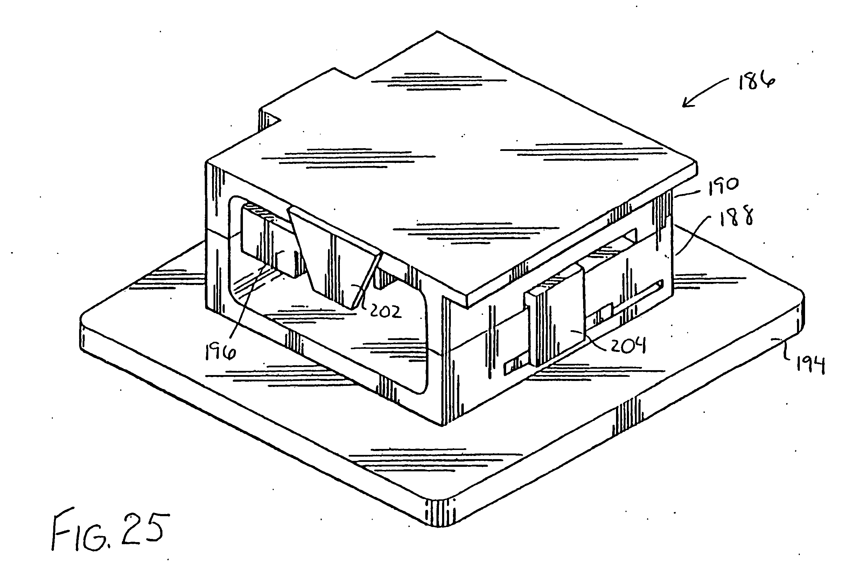

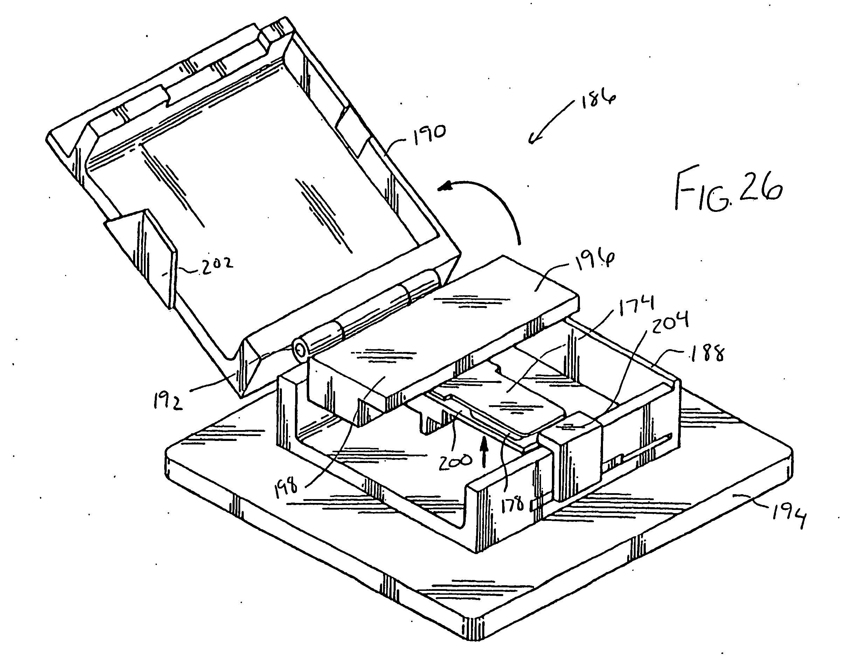

Referring to Figs. 25 and 26, another embodiment of a powder conditioning device 186 will be described. Similar to powder conditioning device 160, device 186 may function as a package that is used to hold a receptacle until ready for use. Device 186 comprises a base 188 and a cover 190 that is pivotally coupled to base 188 by a hinge 192. Optionally, base 188 may be coupled to a platform 194.

Also pivotally coupled to base 188 by hinge 192 is a coupling arrangement 196. Coupling arrangement 196 includes a holder 198 to which receptacle 174 is coupled. Also coupled to coupling arrangement 196 is a cantilever beam 200. In this way, cantilever beam 200 will pivot along with coupling arrangement 196 (which in turn holds receptacle 174). Attached to cover 190 is an arm 202 that includes a projecting tab (hidden from view) that is configured to engage coupling arrangement 196 as cover 190 is moved to an open position as shown in Fig. 26. Slidably coupled to base 188 is a latch 204 that extends partially across cantilever beam 200. To access receptacle 174, a user opens cover 190, and the projecting tab on arm 202 causes coupling arrangement 196 to rotate. In so doing, receptacle 174 also rotates while latch 204 engages cantilever beam 200. Upon further rotation of cover 190, cantilever beam 200 becomes disengaged from latch 204 and springs upward to strike cavity 178 of receptacle 174. The amount of energy supplied to the powder may be controlled depending on the stiffness of cantilever beam 200 and/or the configuration of receptacle 174. Following conditioning of the powder in receptacle 174, receptacle 174 may be removed from coupling arrangement 196, e.g., by tearing receptacle 174 along score lines. Receptacle 174 may then be placed into an aerosolization device.

Latch 204 is slidably coupled to base 188 to facilitate the final fabrication of device 186. More specifically, after receptacle 174 has been inserted and cover 190 is closed, latch 178 may be moved to the position shown in Fig. 26 so that it is located over cantilever beam 200. As with other embodiments, coupling arrangement 196 may be employed to hold multiple receptacles and multiple latches may be used to condition the powder in each of the receptacles upon opening of cover 190.

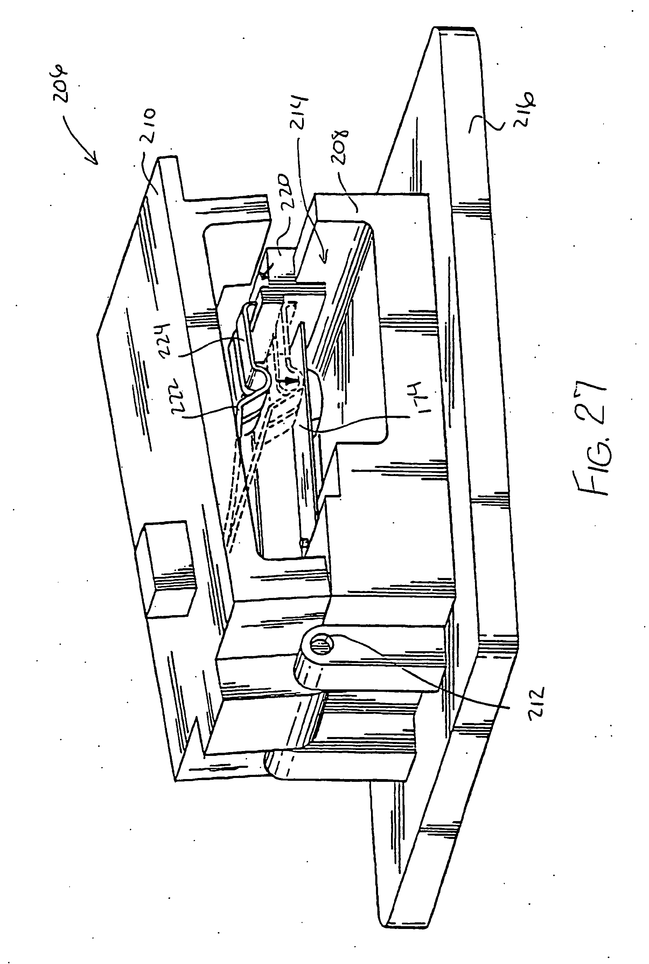

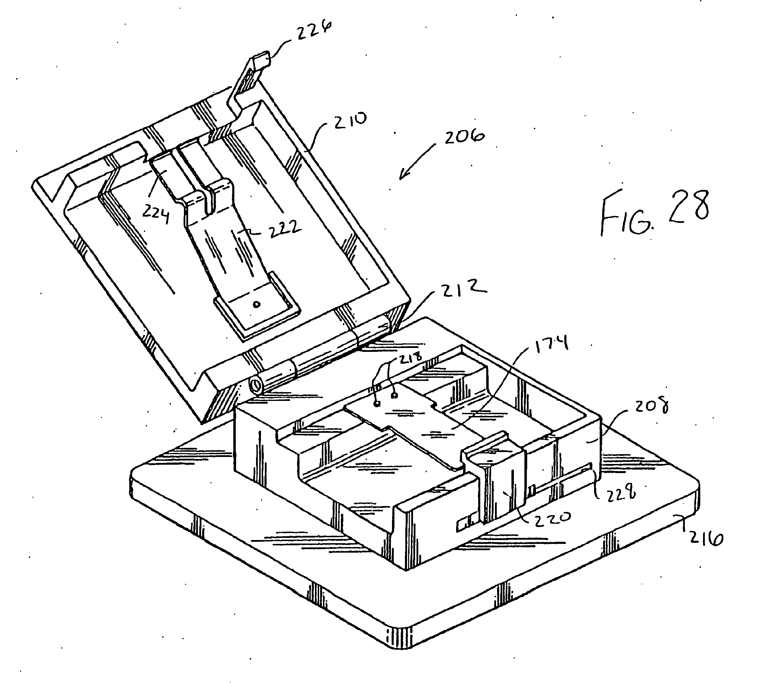

Referring to Figs. 27 and 28, yet another embodiment of a powder conditioning device 206 will be described. Similar to powder conditioning devices 160 and 186, device 206 may be used as packaging when storing and shipping a receptacle. After the receptacle has been removed, device 206 may be discarded. Device 206 comprises a base 208 and a cover 210 that is pivotally coupled to base 208 by a hinge 212. When in a closed position, cover 210 and base 208 define an enclosure 214 into which receptacle 174 is held. Optionally, base 208 may be coupled to a platform 216. Conveniently, base 208 includes pins 218 to properly align receptacle 174 when coupled to base 208.

Slidably coupled to base 208 is a latch 220, and a spring 222 is coupled to cover 210. As best shown in Fig. 27, after receptacle 174 has been mounted to base 208, cover 210 is closed. In so doing, an end 224 of spring 222 engages latch 220. As cover 210 is further closed, spring 222 is forced upward toward cover 210 to "load" the spring. When fully closed, a catch 226 engages a notch 228 to hold cover 210 in the closed position.

When the user is ready to use receptacle 174, latch 220 is slid relative to base 208 to disengage latch 220 from end 224. In so doing, spring 220 springs downward as shown in Fig. 27 to strike receptacle 174. In so doing, the powder within receptacle 174 is conditioned. Cover 210 may then be moved to the open position as shown in Fig. 28 and receptacle 174 removed from enclosure 214. The receptacle may then be placed into an aerosolization device for aerosolization. As with other embodiments, it will be appreciated that multiple receptacles may be held within enclosure 214 to simultaneously condition the powder in multiple receptacles. Further, the amount of energy supplied to the powder may be varied by varying the spring constant of spring 222.

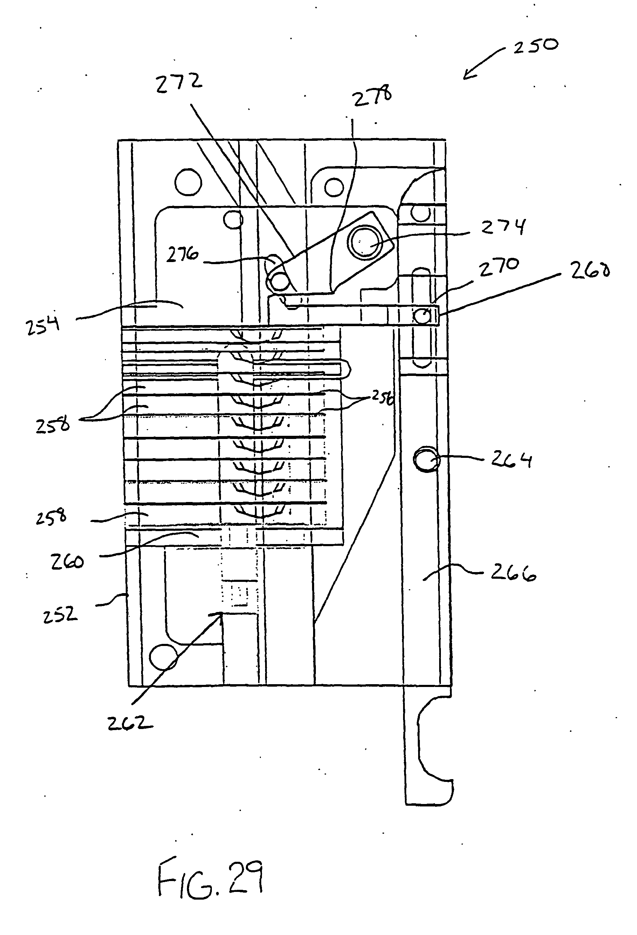

Referring now to Fig. 29, another embodiment of a powder conditioning device 250 will be described. Device 250 comprises a housing 252 having an interior 254 for storing multiple stacked receptacles 256 that each hold an amount of powder that is to be aerosolized by a separate aerosolization device. Each receptacle 256 is held on a spacer 258 that separates the receptacles from each other. The bottom spacer 258 rests on top of an index plate 260 of an advancement mechanism 262 which utilizes a spring or other biasing mechanism (not shown) to apply an upward force to receptacles 256. In this way, as one of the receptacles is removed from housing 252, the receptacles 256 are advanced upward so that the next receptacle may be treated as described hereinafter.

Pivotally coupled to housing 252 by a pivot pin 264 is a trigger 266. A push plate 268 is pivotally coupled to trigger 266 by a pivot pin 270. As shown in Fig. 29, trigger 266 is in a home position where push plate 268 prevents receptacles 256 from indexing. Push plate 268 is also employed to prevent the downward release of a spring biased strike plate 272. Strike plate 272 is pivotally coupled to housing 252 by a pivot pin 274 and is configured to rotate within a slot 276. A spring (not shown) is used to bias strike plate 272 against push plate 268.

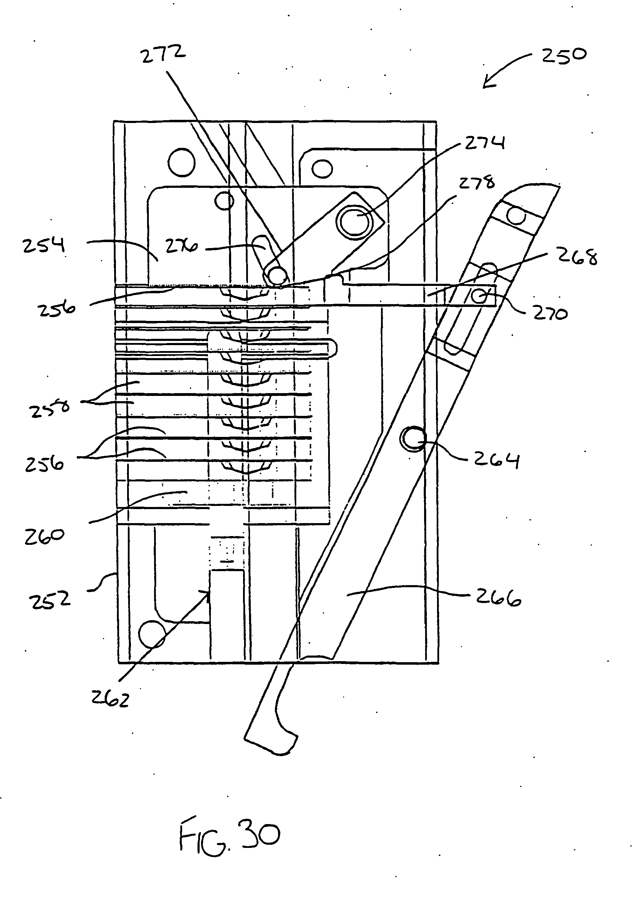

When a user is ready to remove one of the receptacles 256, trigger 266 is pulled to a dispensing position as shown in Fig. 30. In so doing, push plate 268 is moved past the top receptacle to permit advancement mechanism 262 to move the stack of receptacles 256 upward. A fraction of a second after the receptacles begin to advance, push plate 268 passes a drop point 278 on strike plate 272. As the drop point 278 is passed, strike plate 272 pivots downward by the force of the spring to strike the top receptacle 256 (just as the top receptacle is moving upwards). Hence, by pulling trigger 266 to the dispensing position, the top receptacle is provided with a pulse of energy to condition its powder prior to removal from housing 252.

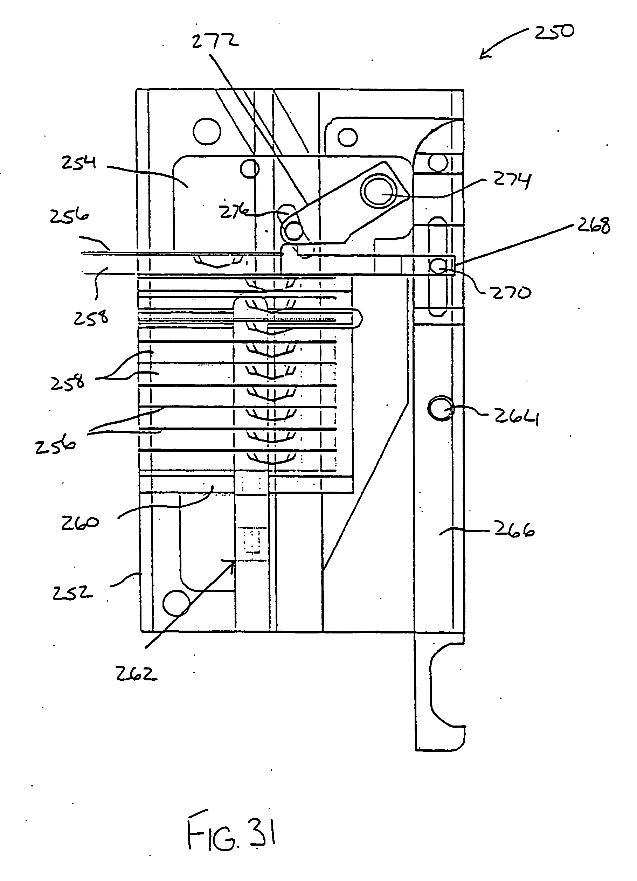

As shown in Fig. 31, trigger 266 is moved back to the home position, causing push plate 268 to push the top receptacle (which has been indexed upward by advancement mechanism 262) partially out of housing 252. The user may then grasp the dispensed receptacle, remove it from its spacer 258, and place the receptacle into an aerosolization device for aerosolization of the powder. With trigger 266 back in the home position, the next receptacle in the stack may be treated and dispensed by repeating the same process.

The invention has now been described in detail for purposes of clarity and understanding. However, it will be appreciated that certain changes and modification may be practiced within the scope of the appended claims.

## Claims

1. An aerosolization device comprising:
a receptacle (10) having an enclosed chamber (18) containing a powder; and
an aerosolization mechanism (70) to aerosolize the powder in the receptacle (10) by flowing gas through the chamber (18) by flowing gas into and out of the chamber (18),
**characterized in that** the device further comprises:
a mechanism (22, 60, 100, 106, 108, 120, 160, 186, 206, 250) adapted to provide at least one pulse of energy to the receptacle prior to gas flowing through the chamber to increase the efficiency at which the powder may be extracted from the chamber when the gas flows through the chamber (18).

2. A device as in claim 1 wherein the mechanism that provides at least one pulse of energy to the receptacle comprises a striking device (22, 60, 120, 160, 186, 206, 250) to quickly strike the receptacle (18).

3. A device as in claim 2 wherein the striking device (22, 60, 120, 160, 186, 206, 250) is configured to strike the receptacle with at least about 0.01 lbf-in in energy.

4. A device as in claim 2 wherein the striking (22, 60, 120, 160, 186, 206, 250) device comprises a spring-loaded lever arm (34, 84, 126).

5. A device as in claim 1 wherein the mechanism to provide at least one pulse of energy to the receptacle comprises a pivotal latch having a lock that pivots as the receptacle is moved against the latch, and a trigger having a ramp, wherein the lock of the latch is slidable upon the ramp when the latch pivots to cause the lever arm to pivot and compress a first spring and cause the lock to engage the trigger to lock the lever arm in an energy storage position.

6. A device as in claim 5 further comprising a second spring that is in contact with the trigger, wherein further movement of the receptacle causes the receptacle to engage and move the trigger away from the lock to release the lever arm which then strikes the receptacle.

7. A device as in claim 1 wherein the mechanism to provide at least one pulse of energy to the receptacle comprises a bending device that is configured to bend and then quickly release the receptacle to permit the receptacle to strike a surface.

8. A device as in claim 1 wherein the mechanism to provide at least one pulse of energy to the receptacle comprises a vibratable element that is configured to at least temporarily contact the receptacle.

9. A device as in claim 1 wherein the powder is composed of fine particles having a mean size in the range from about 0.5 µm to about 5 µm.

10. A device as in claim 1 wherein the receptacle further comprises a metallic body having a tab extending from the chamber.

11. A device as in claim 1 wherein the aerosolization mechanism provides a vacuum to flow gas through the chamber.

## Patentansprüche

1. Aerosolisierungsvorrichtung, die aufweist:
ein Gefäß (10) mit einer abgeschlossenen Kammer (18), die ein Pulver enthält; und
einen Aerosolisierungsmechanismus (70), um das Pulver im Gefäß (10) zu aerosolisieren, indem Gas durch die Kammer (18) strömen gelassen wird, indem Gas in die und aus der Kammer (18) strömen gelassen wird,
**dadurch gekennzeichnet, daß** die Vorrichtung ferner aufweist:
einen Mechanismus (22,60,100,106,108,120,160,186,206,250), der eingerichtet ist, dem Gefäß mindestens einen Energieimpuls zuzuführen, bevor Gas durch die Kammer strömt, um die Effizienz zu erhöhen, mit der das Pulver aus der Kammer extrahiert werden kann, wenn das Gas durch die Kammer (18) strömt.

2. Vorrichtung nach Anspruch 1, wobei der Mechanismus, der dem Gefäß mindestens einen Energieimpuls zuführt, eine Schlagvorrichtung (22,60,120,160,186,206,250) aufweist, um schnell auf das Gefäß zu schlagen.

3. Vorrichtung nach Anspruch 2, wobei die Schlagvorrichtung (22,60,120,160,186,206,250) konfiguriert ist, mit mindestens etwa 0.01 lbf-in Energie auf das Gefäß zu schlagen.

4. Vorrichtung nach Anspruch 2, wobei die Schlagvorrichtung (22,60,120,160,186,206,250) einen federbelasteten Hebelarm (34,84,126) aufweist.

5. Vorrichtung nach Anspruch 1, wobei der Mechanismus, um dem Gefäß mindestens einen Energieimpuls zuzuführen, eine Drehklinke mit einer Arretierung, die sich dreht, wenn das Gefäß gegen die Klinke bewegt wird, und einen Auslöser mit einer Rampe aufweist, wobei die Arretierung der Klinke auf der Rampe verschiebbar ist, wenn sich die Klinke dreht, um zu bewirken, daß sich der Hebelarm dreht und eine erste Feder zusammendrückt, und um zu bewirken, daß die Arretierung mit dem Auslöser in Eingriff tritt, um den Hebelarm in einer Energiespeicherposition zu arretieren.

6. Vorrichtung nach Anspruch 5, die ferner eine zweite Feder aufweist, die mit dem Auslöser in Kontakt steht, wobei eine weitere Bewegung des Gefäßes bewirkt, daß das Gefäß mit dem Auslöser in Eingriff tritt und ihn von der Arretierung weg bewegt, um den Hebelarm freizugeben, der dann auf das Gefäß schlägt.

7. Vorrichtung nach Anspruch 1, wobei der Mechanismus, um dem Gefäß mindestens einen Energieimpuls zuzuführen, eine Biegevorrichtung aufweist, die konfiguriert ist, sich zu biegen und das Gefäß dann schnell freizugeben, um es zu ermöglichen, daß das Gefäß auf eine Oberfläche schlägt.

8. Vorrichtung nach Anspruch 1, wobei der Mechanismus, um dem Gefäß mindestens einen Energieimpuls zuzuführen, ein schwingungsfähiges Element aufweist, das konfiguriert ist, mindestens zeitweilig das Gefäß zu berühren.

9. Vorrichtung nach Anspruch 1, wobei das Pulver aus feinen Teilchen besteht, die eine mittlere Größe im Bereich von etwa 0,5 µm bis etwa 5 µm aufweisen.

10. Vorrichtung nach Anspruch 1, wobei das Gefäß ferner einen Metallkörper mit einer Nase aufweist, die sich von der Kammer erstreckt.

11. Vorrichtung nach Anspruch 1, wobei der Aerosolisierungsmechanismus einen Unterdruck bereitstellt, um Gas durch die Kammer strömen zu lassen.

## Revendications

1. Dispositif de production d'aérosol comprenant :
→ un récipient (10) ayant une chambre fermée (18) contenant une poudre ; et
→ un mécanisme de production d'aérosol (70) pour pulvériser la poudre dans le récipient (10) en laissant s'écouler du gaz à travers la chambre (18), en laissant s'écouler du gaz à l'intérieur et à l'extérieur de la chambre (18),
**caractérisé en ce que** le dispositif comprend en outre :
un mécanisme (22, 60, 100, 106, 108, 120, 160, 186, 206, 250) adapté pour fournir au moins une impulsion d'énergie au récipient avant que le gaz ne s'écoule à travers la chambre afin d'augmenter le rendement auquel la poudre peut être extraite de la chambre lorsque le gaz s'écoule à travers la chambre (18).

2. Dispositif selon la revendication 1, dans lequel le mécanisme qui fournit au moins une impulsion d'énergie au récipient comprend un dispositif de frappe (22, 60, 120, 160, 186, 206, 250) afin de frapper rapidement le récipient (18).

3. Dispositif selon la revendication 2, dans lequel le dispositif de frappe (22, 60, 120, 160, 186, 206, 250) est configuré pour frapper le récipient avec au moins une énergie d'environ 0,01 livre-force - pouce.

4. Dispositif selon la revendication 2, dans lequel le dispositif de frappe (22, 60, 120, 160, 186, 206, 250) comprend un bras de levier à ressort (34, 84, 126).

5. Dispositif selon la revendication 1, dans lequel le mécanisme pour fournir au moins une impulsion d'énergie au récipient comprend un loquet pivotant ayant un dispositif d'arrêt qui pivote lorsque le récipient est déplacé contre le loquet, et un déclencheur ayant une rampe, dans lequel le dispositif d'arrêt du loquet peut coulisser sur la rampe lorsque le loquet pivote pour amener le bras de levier à pivoter et à comprimer un premier ressort et amener le dispositif d'arrêt à mettre en prise le déclencheur afin de bloquer le bras de levier dans une position de stockage d'énergie.

6. Dispositif selon la revendication 5, comprenant en outre un second ressort qui est en contact avec le déclencheur, dans lequel le mouvement supplémentaire du récipient amène le récipient à mettre en prise et à éloigner le déclencheur du dispositif d'arrêt pour libérer le bras de levier qui frappe alors le récipient.

7. Dispositif selon la revendication 1, dans lequel le mécanisme pour fournir au moins une impulsion d'énergie au récipient comprend un dispositif de flexion qui est configuré pour fléchir et libérer ensuite rapidement le récipient afin de permettre au récipient de frapper une surface.

8. Dispositif selon la revendication 1, dans lequel le mécanisme pour fournir au moins une impulsion d'énergie au récipient comprend un élément pouvant vibrer qui est configuré pour être en contact au moins temporairement avec le récipient.

9. Dispositif selon la revendication 1, dans lequel la poudre est composée de fines particules ayant une taille moyenne de l'ordre d'environ 0,5 µm à environ 5 µm.

10. Dispositif selon la revendication 1, dans lequel le récipient comprend en outre un corps métallique ayant une languette s'étendant à partir de la chambre.

11. Dispositif selon la revendication 1, dans lequel le mécanisme de production d'aérosol fournit un vide pour laisser s'écouler du gaz à travers la chambre.
